# EUROPEAN PATENT APPLICATION

(11) **EP 1 859 794 A1**
(43) Date of publication of application: **28.11.2007**
(21) Application number: 07251704.8
(22) Date of filing: 24.04.2007
(51) Int. Cl.: A61K 31/195, A61K 9/00, A61K 9/20

(54) **Sustained release supplements**

(30) Priority: 24.04.2006 US 794588 P
(71) Applicant: Golini, Jeffrey M., Billings MT 59105 (US)
(72) Inventor: Golini, Jeffrey M., Billings MT 59105 (US)
(74) Representative: Brown, David Leslie

(57) **Abstract**

The invention relates to oral sustained release composition comprising creatine or a creatine salt or ester. The invention includes compositions with one or more excipients, where at least one excipient is capable of delaying release of the creatine. The invention also includes oral dosage forms having an enteric coating.

## Description

### TECHNICAL FIELD

The present invention relates to oral creatine supplements, and methods for making them.

### BACKGROUND ART

Taking creatine orally has been used to increase creatine phosphate stores in the human body. This is important because creatine aids in the process of creating energy usable by muscles of the athlete.

When an athlete exercises or tenses a muscle, energy is required for the muscle to function properly. The energy it uses comes from several different sources, but primarily from nutrients obtained from food. These nutrients are broken down by natural processes occurring within the human body, and new compounds formed which are used to develop energy used by muscles. One of these compounds is adenosine triphosphate (ATP). When muscle energy is needed this ATP is broken down one step further into a chemical called adenosine diphosphate (ADP). This process releases energy which is then used by the contracting muscles. Without sufficient ATP, muscles do not perform properly.

Conventional creatine on the market utilize a quick release delivery via capsules, liquids or powders while other known energy stimulants have only superficially energized the body, and do not increase the body's ability to produce its own ATP stores.

Muscle can store only limited amounts of ATP. As a result, it has been found that with about 5-10 seconds of muscle exertion, the amount of stored ATP is depleted. This results in muscle failure and fatigue. When this happens, the body tries to restore its immediate source of ATP by borrowing a high energy phosphate from a chemical called creatine phosphate (CP). Muscle cells store the chemical, CP, in the same way it stores ATP. If high intensity exercise goes beyond 10 seconds, the body will continue to try and restore its ATP levels by a process called glycolysis. This process is complicated and is a slow method of restoring ATP levels. This is a special problem for anaerobic athletes who require instant energy to maintain and sustain high powered muscle contractions.

By orally supplementing with creatine, an athlete can enhance his body's storage levels of CP. As the muscle runs out of ATP, it can recharge itself by borrowing this CP molecule. Research has shown that by supplementing with creatine, 4-6 times a day for two or more days, the human body showed a significant increase in total creatine phosphate concentration.

ATP or CP are unstable and cannot be ingested directly. First it is expensive, most is destroyed by oxygen and the rest in the stomach. However, it has been found that creatine can be ingested and converted by the body to CP. The known methods of ingesting creatine utilize a quick release delivery.

An object of the invention is to provide an improved composition for oral delivery of creatine.

### DISCLOSURE OF THE INVENTION

In one aspect the invention provides an oral sustained release composition comprising creatine, or a creatine salt or ester together with one or more excipients, wherein at least one excipient is capable of delaying release of the creatine.

In another aspect the invention provides an oral dosage form having an enteric coating, the dosage form comprising creatine or a creatine salt or ester.

For a better understanding of the invention, the following terms shall have the meaning as set forth below:
Creatine: Any form of a creatine that is taken orally, such as creatine monohydrate, creatine citrate, creatine malate, creatine ethyl ester, creatine titrate, creatine glutamate, creatine alpha ketoglutarate, creatine phosphate, creatine chelate, etc.
*Excipients:* A more or less inert substance added to an active item as a dilutent, compressing agent, filler, flow agent, coating, delivery vehicle or to give form or consistency. Basically any compound forming a part of the formulation that is not an active ingredient Excipients are selected that are pharmaceutically acceptable.
*Quick* Release: Delivery system for dispensing an active compound all at once or over a short time period. The following are typical quick release break down times for various delivery systems:

| | | |
|---|---|---|
| 1) | Powder | Zero time once it hits the stomach. Immediate Release. |
| 2) | Liquid | Zero time once it hits the stomach. Immediate Release. |
| 3) | Softgel | Approximately 15 minutes for the gelatin to break and the active to be released. |
| 4) | Tablet | Approximately 5 - 15 minutes for the tablet to break apart and 100% of the actives to be released. |
| 5) | Capsule | Approximately 15 minutes for the gelatin to break apart and 100% of the actives to be released. |

*Sustained* Release: Delivery system for dispensing an active compound over a long period of time. Other terms that describe this delivery system are controlled release, time release, slow release, continuous release, delayed release, gradual release, space release, extended action, layered time action, prolonged release or action, modified release, extended release, etc. Specifically, sustained release allows for delivery of the active compound over a period of time longer than the times set for the quick release systems as set forth below. Preferably the sustained release is from an enteric formulation.

Comprising: The term "comprising" as used in this specification means "consisting at least in part of". When interpreting each statement in this specification that includes the term "comprising", features other than that or those prefaced by the term may also be present. Related terms such as "comprise" and "comprises" are to be interpreted in the same manner.

Excipients that may be used in the invention to provide sustained release of creatine are preferably in the form of a matrix. The matrix may comprise one or more polymers.

The polymer may be a water-soluble hydrophilic polymer, or a water insoluble hydrophobic polymer (including waxes). Examples of suitable water soluble polymers include polyvinylpyrrolidine, hydroxypropyl cellulose, hydroxypropylmethyl cellulose, methyl cellulose, vinyl acetate copolymers, polysaccharides (such as alignate, xanthum gum, etc.) polyethylene oxide, methacrylic acid copolymers, maleic anhydride/methyl vinyl ether copolymers and derivatives and mixtures thereof. Examples of suitable water insoluble hydrophobic polymers include acrylates, cellulose derivatives such ethylcellulose or cellulose acetate, polyethylene, methacrylates, acrylic acid copolymers and high molecular weight polyvinylalcohols. Examples of suitable waxes include fatty acids and glycerides.

Preferably, the polymer is selected from hydroxypropyl cellulose, hydroxypropylmethyl cellulose, and methyl cellulose. More preferably, the polymer is hydroxypropylmethyl cellulose.

A particularly preferred polymer for use in the invention is polyvinyl acetate phthalate.

The matrix formulations are generally prepared using standard techniques well known in the art. Typically, they are prepared by dry blending the polymer, filler, creatine, and other excipients followed by granulating the mixture using an alcohol until proper granulation is obtained. The granulation is done by methods known in the art. The wet granules are dried in a fluid bed dryer, sifted and ground to appropriate size. Lubricating agents are mixed with the dried granulation to obtain the final formulation.

The composition of the invention also typically includes pharmaceutically acceptable excipients. As is well known to those skilled in the art, pharmaceutical excipients are routinely incorporated into solid dosage forms. This is done to ease the manufacturing process as well as to improve the performance of the dosage form. Common excipients include diluents or bulking agents, lubricants, binders, etc. Such excipients are routinely used in the dosage forms of this invention.

Diluents, or fillers, are added in order to increase the mass of an individual dose to a size suitable for tablet compression. Suitable diluents include powdered sugar, calcium phosphate, calcium sulfate, microcrystalline cellulose, lactose, mannitol, kaolin, sodium chloride, dry starch, sorbitol, etc.

Lubricants are incorporated into a formulation for a variety of reasons. They reduce friction between the granulation and die wall during compression and ejection. This prevents the granulate from sticking to the tablet punches, facilitates its ejection from the tablet punches, etc. Examples of suitable lubricants include talc, stearic acid, vegetable oil, calcium stearate, zinc stearate, magnesium stearate, etc.

Glidants are also typically incorporated into the formulation. A glidant improves the flow characteristics of the granulation. Examples of suitable glidants include talc, silicon dioxide, and cornstarch.

Binders may be incorporated into the formulation. Binders are typically utilized if the manufacture of the dosage form uses a granulation step. Examples of suitable binders include., povidone, polyvinylpyrrolidone, xanthan gum, cellulose gums such as carboxymethylcellulose, methyl cellulose, hydroxypropylmethylcellulose, hydroxycellulose, gelatin, starch, and pregelatinized starch.

Other excipients that may be incorporated into the formulation include preservatives, antioxidants, or any other excipient commonly used in the pharmaceutical industry, etc.

Any suitable enteric coating may be used. The coating should withstand the acid conditions of the stomach but release the contents in the small intestine. The coating liquid generally comprises film forming polymers such as hydroxypropyl cellulose, hydroxypropylmethyl cellulose, cellulose esters or ethers such as cellulose acetate or ethylcellulose, an acrylic polymer or a mixture of any of these polymers. The coating solution is generally an aqueous solution or an organic solvent further comprising propylene glycol, sorbitan monoleate, sorbic acid, fillers such as titanium dioxide, a pharmaceutically acceptable dye.

Polyvinyl acetate phthalate is a preferred coating material. Tablets may be coated directly or alternatively gelatine capsules comprising creatine may be coated.

The creatine formulation according to the present invention is used for ingestion and may include tablets, softgels, caplets, suspensions, etc. The invention also includes tablets, capsules and crystals that are enteric coated.

Forms of the invention with enteric coatings are particularly preferred. Creatine is unstable in the stomach and rapidity converts to creatinine, a very dangerous bio-waste. A need is present for a way to protect the creatine from the harsh acidic stomach acids. By developing a sustained released tablet that does not break down in acid, but will pass through the stomach and into the intestines, while only breaking down with an alkaline, this can be accomplished.

Typically, the creatine formulations will contain about 40-90% (w/w), preferably 50 - 70% creatine and the remainder excipients. The amount of active creatine can vary based on gender, age and activity levels. This formulation is characterized by protecting the creatine from the stomach and conversion to creatinine from chemical reaction. The formulations can be such a sustained release, which will withstand gastric fluids at low pH and not disperse until reaching the alkaline pH of the intestines, completely bypassing breakdown in the stomach. Typically the dose of creatine administered to a human is 50-2000 mg of creatine as creatine or as part of a creatine salt. Oral dosage forms generally comprise 100-1000 mg most preferably 250-750 mg.

In a further aspect the invention provides the use of creatine in the manufacture of an oral sustained release composition or a dosage form for providing sustained bioavailability of creatine for anaerobic exercise.

### BRIEF DESCRIPTION OF THE DRAWINGS

In order that the invention may be clearly understood and readily carried into effect, preferred embodiments of the invention will now be described, by way of example only, with reference to the accompanying drawings wherein:

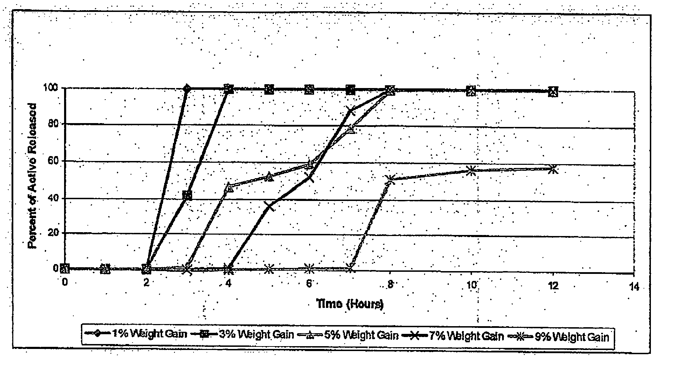

Fig. 1 is in a graph showing the percent of active creatine released over time for various enteric coated tablets.

### EXAMPLES

The following examples further illustrate practice of the invention.

### EXAMPLE 1

**Tablet (dry granulation and compression):**

| | | |
|---|---|---|
| | Creatine | 500 mg |
| | Microcrystalline Cellulose: | 70 mg |
| | Hydroxypropyl Methylcellulose | 120 mg |
| | Magnesium Stearate | 5 mg |
| | Hydroxypropyl Cellulose | 10 mg |
| | Sorbitol | 150 mg |
| | Polyvinyl acetate phthalate | 20 mg |

| Soft Gel: | | |
|---|---|---|
| | Creatine | 375 mg |
| | soy bean oil | 100 mg |
| | water | 50 mg |
| | glycerine | 50 mg |
| | lecithin | 75 mg |
| | bees wax | 100 mg |
| | Gelatin | 400 mg |
| | Polyvinyl acetate phthalate | 20 mg |

| Capsule: | | |
|---|---|---|
| | Creatine | 500 mg |
| | Enteric Coating | 25 mg |
| | Gelatin Capsule | 125 mg |
| | Polyvinyl acetate phthalate | 20 mg |

| Powder. | | |
|---|---|---|
| | Creatine | 500 mg |
| | Polyvinyl acetate phthalate | 20 mg |

Table 1 is data showing the physical characteristics of a sustain release oral tablet using Kre-Alkalyn^{®} creatine. Physical testing was carried out using a Erweka MultiCheck direct measurement apparatus.

**Table 1**

| | |
|---|---|
| Weight | 1400 mg |
| Thickness | 10.22 mm |
| Hardness | 25.5 kp |
| Diameter | 19.10 mm |
| Friability | 0.008% |

### EXAMPLE 2 DISSOLUTION OF ORAL TABLET

Fig. 1 is a graph showing dissolution of a sustain release oral tablet using Kre_Alkalyn^{®} creatine and the excipients as set forth in the Example 1. The graph shows the delivery time for tablets having different thicknesses of an enteric film coat mix comprising a mixture of 60% PV enteric and 40% AP enteric (Color Con). The tablets are spray coated with this mixture and the graph shows the results when the tablets are spray coated with a percentage by tablet weight of enteric mixture. The graph shows the results with a 1%, 3%, 5%, 7% and 9% by tablet weight of enteric coating. Dissolution was carried out using a USP apparatus I at 37.5° C and 100 rpms. As can be seen, the time for releasing the creatine may be controlled by varying the percentage by tablet weight of the enteric coating applied.

### Timed Release Action comes from:

1) Tablet Excipients which hold the tablet together
2) Film coating which does not break down at a low pH in the stomach, but is released in the intestines under higher pH conditions.

Sustained Release 60/40 PV/AP Film Coat Mix: This is a 60% PV Enteric coat mixed with a 40% AP Enteric coat film mix. The Time Released Tablet is spray coated with this mixture, which is a typical mixture but many more combinations can be used to achieve the same results.

1% weight means we added 1% by tablet weight or 8.75 mg of the PV/AP. This amount was spray dried on the tablet to coat it.

2%, 5%, 7% and 9%. The more coating the longer it took to break down. See Table 1 below.

**TABLE 1**

| % Film Coat | Active released in 2 hours | Active released in 3 hours | Active released in 6 hours | Active released in 8 hours | Active released in 12 hours |
|---|---|---|---|---|---|
| 1% | 0% | 100% | | | |
| 3% | 0% | 40% | | | |
| | | 100% by 3.5 hours | | | |
| 5% | 0% | 0% | 60% | 100% | |
| 7% | 0% | 0% | 52% | 100% | |
| 9% | 0% | 0% | 0% | 55% | 100% |

### EXAMPLE 3 STABILITY OF CREATINE PRODUCTS IN ACIDIC CONDITIONS

Table 2 shows that creatine is very unstable at pH.

**TABLE 2 Change in creatine with time at pH 4**

| Contact duration (min) | Result | Unit |
|---|---|---|
| 0.25 | 0.94 | relative wt |
| 0.50 | 0.86 | relative wt |
| 0.75 | 0.75 | relative wt |
| 1.00 | 0.66 | relative wt |
| 1.25 | 0.57 | relative wt |
| 1.50 | 0.49 | relative wt |
| 1.75 | 0.38 | relative wt |
| 2.00 | 0.31 | relative wt |
| 2.25 | 0.21 | relative wt |
| 2.50 | 0.16 | relative wt |
| 2.75 | 0.06 | relative wt |
| 3.00 | 0.005 | relative wt |

The determination was based upon formation of creatinine under buffered incubation conditions. A solution of 1% creatine in simulated gastric solution (0.5% betain/pepsin in 1M HEPES buffer pH controlled with 5M HC1 and 5N NaOH). Creatinine determination performed by PDA spectrophotometer in kinetic mode, scanning at 15 sec intervals with an integration time of 0.5 sec. Detection in 1 cm path length flow call at 37°C with test analyte flowing at 1 ml/min. Reagents obtained from Sigma-Aldrich.

Tests on the coated Tablet prepared as described in Example 2 but with addition of 6% by tablet weight of PV/AP, creatine monohydrate powder and capsules of creatine monohydrate were carried out

These tests were done on an "AS IS or DRY BASIS.

### Testing Method:

### FTNIR Analysis by:

Identification performed by FINIR, against in-house external library standards obtained from Sigma & produced by HPLC. / Quantification by FTNIR against external library standards obtained from Sigma & produced by HPLC

### Testing Equipment:

Bran & Luebbe InfraProver II FTNIR

### HPLC Analysis by:

Analysis performed by HPLC using Intersil ODS-2 5um (250x4.dmm) and 25 min. gradient elution with 0.1% phosphoric acid buffer in H₂O and 0.1% phosphoric acid in acetonitrile.
External reference standards obtained from Sigma-Aldrich.

### Results:

| | | |
|---|---|---|
| Creatine Capsule (quick release gelatin capsule) | Creatine 99.9% | Creatinine <200ppm |
| Creatine Powder | Creatine 100.1% | Creatinine <200ppm |
| Creatine Sustained Released Tablet (coated as in Example 3) | Creatine 99.7% | Creatinine <200ppm |

### Step 2:

1 capsule of creatine (500 mg) and 500 mg of powder was used along with 1 tablet of 500 mg of Sustained Released Creatine.

### Step 3:

All 3 products were placed in a stomacher at pH 3 for 60 minutes to simulate digestion. Stomacher contained 1 liter of fluid with pH being adjusted with hydrochloric acid to simulate digestion.

### Step 4:

After 60 minutes of digestion simulation in stomacher, all 3 products were tested for:
Total Creatine levels
Total Creatinine levels

### Results:

| **Product** | **Amount Used: Elemental Creatine** | **pH of Stomacher after addition of product** | **Total conversion to Creatinine after 60 minutes of digestion** |
|---|---|---|---|
| **Creatine Capsule** | **500 mg** | **3.6** | **100%** |
| **Creatine Powder** | **500 mg** | **3.5** | **100%** |
| **Creatine Sustained Released Tablet** | **500 mg** | **3.5** | **0%** |

| | | | |
|---|---|---|---|
| ****Sustained Released Tablet was still 100% intact and unaffected by the stomach acid.** | | | |

### 1). NIR Analysis of stomach:

*500 mg creatine capsule was added to stomacher at pH 3, raised stomach pH level to 3.6, with all creatine being converted to creatinine after 60 minutes. The capsule dissolved and the powder dispersed after 15 minutes.

*500 mg creatine powder was added to stomacher at pH 3, raised stomach pH level to 3.5, with all creatine being converted to creatinine after 60 minutes.

*500 mg creatine sustained released tablet was added to stomacher at pH 3, raised stomach pH level to 3.5. The tablet stayed completely intact, protecting the creatine from converting to creatinine in the stomach. After testing, the original analysis was duplicated. Creatine 99.7% Creatinine <200ppm

### Conclusions:

Creatine will convert to creatinine in the stomach unless protected by an enteric coating, which prevents the tablet containing the creatine from breaking down in the harsh acids of the stomach. The tablet will pass into the alkaline base intestines where the special coating will allow the tablet to break apart and be absorbed.

### EXAMPLE 4 EFFECT OF SUSTAINED RELEASE CREATINE

Week 1-4: Two subjects were administered creatine monohydrate in powder form for four weeks. 2 grams were taken in the A.M. before breakfast.

No changes were made to diet and athletes were working out 4 times per week.

Stamina & Strength levels were monitored at the beginning of the study and every workout for 4 weeks.

A combination of free weights and hammer strength machines were used.

Both subjects did not notice any stamina increase. They were able to do about the same amount of repetitions of weights at the beginning of the cycle as they could at the end of the first four weeks. About a 1% increase in strength was noticed, but was not contributed to the addition of creatine to the diet, but just from good old fashioned training.

Both athletes experience side effects. Stomach bloating, bloating of the ankles and wrist, along with diarrhea and loose stools. An over all sense of tiredness set in after week two and lasted through week four.

Week 5-8. Athletes discontinued creatine use. After 1 week the stomach bloat, bloating of the ankles and wrist, along with diarrhea and loose stools disappeared. About a 1% increase in strength was again noticed by the end of week 8, due to old fashioned hard work.

Week 9-12. Athletes were administered one 750 mg sustained released tablet in the a.m. and one 750 mg tablet in the afternoon. The breakdown of these tablets were 9 hours.

After just one week, both athletes noticed increases in their repetitions and strength. At the end of week 12, strength gains were in the neighborhood of 15%. Very little side effects were experienced during this time compared to week 1-4.

### Conclusion:

Creatine delivered in a sustained released tablet works much better than taking creatine in a powder form. Much fewer side effects were noticed in the sustained released tablet compared to the powder form.

Any discussion of documents, acts, materials, devices or the like that has been included in the present specification is solely for the purpose of providing a context for the present invention. It is not to be taken as an admission that any or all of these matters forms part of the prior art base or were common general knowledge in the field relevant to the present invention as it existed before the priority date.

The above examples are illustrations of the practice of the invention. It will be appreciated by those skilled in the art that the invention can be carried out with numerous modifications and variations. For example, the compositions can show variations in the creatine content, the excipients and the sustained release materials.

## Claims

1. An oral sustained release composition comprising creatine or a creatine salt or ester together with one or more excipients, wherein at least one excipient is capable of delaying release of the creatine.

2. A composition as claimed in claim 1 comprising 40-90% creatine.

3. A composition as claimed in claim 1 or claim 2 comprising a hydrophilic or hydrophobic matrix.

4. A composition as claimed in claim 3 wherein the matrix comprises one or more from the group consisting of polyvinylpyrrolidine, hydroxypropyl cellulose, hydroxypropylmethyl cellulose, methyl cellulose, vinyl acetate copolymers, polysaccharides, polyethylene oxide, methacrylic acid copolymers, maleic anhydride/methyl vinyl ether copolymers and derivatives, acrylates, cellulose derivatives, polyethylene, methacrylates, acrylic acid copolymers, high molecular weight polyvinylalcohols, and waxes.

5. A composition as claimed in claim 4 wherein the matrix comprises polyvinyl acetate phthalate.

6. A dosage form as claimed in any one of claims 1-5 wherein the composition is in a form selected from a tablet, softgel, caplet and a suspension.

7. An oral dosage form having an enteric coating, the dosage form comprising creatine or a creatine salt or ester.

8. A dosage form as claimed in claim 7 wherein the dosage form is a capsule, a coated tablet or a coated crystal.

9. A dosage form as claimed in claim 7 or claim 8 comprising 40-90% creatine.

10. A dosage form as claimed in claim 7 or claim 8 wherein the coating comprises a film forming polymer selected from the group comprising hydroxypropyl cellulose, hydroxypropylmethyl cellulose, cellulose esters or ethers, an acrylic polymer or a mixture of any of these polymers.

11. A dosage form as claimed in claim 10 wherein the coating comprises polyvinyl acetate phthalate.

12. The use of creatine in the manufacture of an oral sustained release composition or a dosage form for providing sustained bioavailability of creatine for anaerobic exercise.
